# EUROPÄISCHE PATENTSCHRIFT

(11) **EP 2 654 818 B1**
(45) Veröffentlichungstag und Bekanntmachung des Hinweises auf die Patenterteilung: **21.03.2018**
(21) Anmeldenummer: 11788432.0
(22) Anmeldetag: 23.11.2011
(51) Int. Cl.: A61L 29/04

(54) **POLYAMID/POLYVINYLPYRROLIDON (PA/PVP)-POLYMERGEMISCH ALS KATHETERMATERIAL**
POLYAMIDE/POLYVINYLPYRROLIDONE (PA-PVP) BLEND AS CATHETHER MATERIAL
MELANGE POLYAMIDE/POLYVINYLPYRROLIDONE POUR CATHÉTER

(30) Priorität: 21.12.2010 US 201061425254 P
(43) Veröffentlichungstag der Anmeldung: 30.10.2013
(73) Patentinhaber: Biotronik AG, 8180 Bülach (CH)
(72) Erfinder: QUINT, Bodo, CH-8154 Oberglatt (CH); WESSELMANN, Matthias, CH-8455 Rüdlingen (CH); SAGER, Laura, CH-8038 Zürich (CH)
(74) Vertreter: Randoll, Sören
(86) Internationale Anmeldenummer: PCT/EP2011/070814
(87) Internationale Veröffentlichungsnummer: WO 2012/084390

(56) Entgegenhaltungen:
- EP-A2- 0 697 219
- WO-A1-2006/065905
- DE-A1- 19 933 279

## Beschreibung

Die Angioplastie, auch perkutane transluminale Angioplastie (PTA) oder perkutane transluminale Coronarangioplastie (PTCA), ist ein Verfahren zur Erweiterung oder Wiedereröffnung von verengten oder verschlossenen Blutgefäßen (meistens Arterien, seltener auch Venen). Ein gebräuchliches Verfahren der Angioplastie ist die Ballondilatation.

Unter der Ballondilatation im Rahmen einer Angioplastie versteht man in der interventionellen Radiologie, der Kardiologie und der Angiologie eine Methode zur Aufdehnung krankhaft verengter Blutgefäße mittels eines Ballonkatheters, einem Gefäßkatheter mit daran angebrachtem Ballon, der sich erst an der verengten Stelle langsam unter hohem Druck (6-20 bar) entfaltet. Dadurch werden die Engstellen, die v. a. durch atherosklerotische Veränderungen (Gefäßverkalkung) entstehen, so gedehnt, dass sie den Blutstrom nicht mehr oder weniger stark behindern.

Dabei werden die Ballonkatheter meist von der Leiste aus über einen Führungsdraht und Führungskatheter in die Stenose (Engstelle) platziert und mit Druck aufgeblasen. Hierdurch wird die Engstelle beseitigt und eine Operation vermieden.

Daneben werden Katheter mit einem deflatierbaren Ballon auch zur Platzierung von Stents genutzt. Dazu trägt der Katheter im Bereich des deflatierbaren Ballons einen Stent, der nach Erreichen der gewünschten Stelle im Blutgefäß durch Deflation des Ballons im Gefäß platziert werden kann.

Moderne Methoden im Bereich Kunststoffverarbeitung ermöglichen die Konstruktion und Weiterentwicklung solcher Ballons, um die Qualität individuell auf die Bedürfnisse der Patienten anzupassen. Wichtig sind hierbei die Flexibilität der Ballons sowie ihre Druckfestigkeit.

Polyamide und Peba-Materialien für den Katheterbau basieren meist auf der Polyamid 12 (PA12) Grundstruktur. Dieses Polyamid zeichnet sich durch hohe Festigkeit und Zähigkeit, geringe Wasseraufnahme und damit verbundene Eigenschaftsänderungen sowie durch eine gute Verfügbarkeit der Rohmaterialien aus. PA12 ist ein verbreitetes Kathetermaterial und wird aus Gründen der guten Verformbarkeit gerne als Basismaterial für Katheterballone genutzt. Die praktische Anwendung fordert für Ballonbauteile eine hohe Druckfestigkeit, eine geringe Wanddicke, aber auch eine hohe Weichheit der Konen.

Um bei gegebenem Werkstoff die Eigenschaften des Ballons zu verbessern, werden die Eigenschaften Orientierung und Kristallinität des Materials gezielt beeinflusst. Die Dehnfähigkeit und damit auch Orientierung des Polymers lässt sich durch Zusatzstoffe erhöhen, die die Gleitfähigkeit der Molekülketten aneinander erhöhen und/ oder die Kristallisation des Polymers vor der Verformung reduzieren, wie z. B. durch Einsatz geeigneter Weichmacher und/oder Lösungsmittel.

PA12-basierte Systeme weisen typischerweise einen Glaspunkt von ca. 50°C auf. Temperaturen oberhalb von 50°C werden zur Blasformung der Ballonbauteile genutzt. Um ein Formgedächtnis in diese Bauteile zu prägen, werden z. B. für Faltung und Stentfixierung ein Formzwang und eine Konditionierung oberhalb bzw. um den Glaspunkt genutzt. Prinzipiell ermöglicht eine Erwärmung der blasgeformten Bauteile in den Bereich und über die Glastemperatur hinaus eine Relaxation der durch die plastische Formung eingeprägten Spannungen. Die Relaxation bewirkt zum Beispiel eine Hysterese zwischen den ersten und weiteren, darauffolgenden Druckbelastungen des Ballonbauteiles. Als konkurrierender Effekt schreitet die Kristallisation des Polymers ab 50°C weiter voran.

Da Polyamide bei Strahlensterilisation typischerweise einen Verlust an mechanischer Festigkeit erleiden und deutlich höhere Temperaturen als der Glaspunkt zu starken dimensionellen Veränderungen führen, hat sich die ETO (Ethylenoxid) Sterilisation als typische Sterilisationsmethode für Ballonkatheter etabliert. Die ETO Sterilisationsprozesse werden bei thermischen Bedingungen um 50°C durchgeführt. Wird der Ballonkatheter ETO sterilisiert, stellt diese thermische Belastung in Anwesenheit von Feuchtigkeit das absolute Minimum an Relaxation PA12-basierter Bauteile während der Herstellung des Katheters dar. In der Regel wird das Ballonbauteil durch thermische Methoden so konditioniert, dass bereits nach der Sterilisation reproduzierbare Dimensionen erhalten werden, welche dann auch durch simulierte Alterung und Lagerung nicht mehr deutlich verändert werden. Dies führt allerdings auch dazu, dass die Balloncompliance der ersten Inflation sich mehr oder minder stark von allen nachfolgenden Inflationen unterscheidet, und bei der ersten Inflation steiler ist. Dieser Effekt ist auch mit einer Durchmessererhöhung des Ballonbauteiles verbunden. Da die Compliance dieser Bauteile bei der ersten Inflation bestimmt wird, führen Folgeinflationen des Ballons zu einer gewissen systematischen Überdilatation der Gefäße. Die Durchmesserzunahme der Ballone mit zunehmender Inflationsanzahl und Inflationsintensität ist somit ein sicherheitsrelevantes Qualitätskriterium.

Ungünstigere Gebrauchseigenschaften von PA12 Ballonen, die beispielsweise durch härtere Ballonkonen verursacht werden, können z. B. materialseitig durch den Einsatz eines weicheren Materials vermindert werden - dabei handelt es sich in der Regel um Peba-Typen oder Polymergemische von PA12 mit solchen Peba-Typen. Grundsätzlich bleibt der Glaspunkt von PA12 hierbei aber unverändert.

Die viskoelastischen Eigenschaften von PA12 sind bei diesen Peba-Typen noch stärker ausgeprägt. Diese viskoelastischen Eigenschaften bewirken ab Temperaturen von ca. 50°C einen Schrumpf, der zu einer deutlicheren Änderung des Ballondurchmessers zwischen der ersten und jeder weiteren, darauffolgenden Druckinflation führt. Es werden somit bei dieser Materialoptimierung Kompromisse mit der Präzision des Dilatationsverhaltens geschlossen. Die Präzision in der Dilatation wird in der Regel hierdurch vermindert.

Ballonkatheter aus Polyamid und einer zweiten Weichmacher-Polymerkomponente wie z.B. Polyurethan oder einem elastomeren EthylenCopolymer sind aus WO2006/065905 bzw. EP0697219 bekannt. Die Weiterentwicklung dieser Dilatationsballone ist somit generell durch die Suche nach technischen Lösungen geprägt, welche die Präzision der Dilatation, das Profil des Katheterproduktes und den Erhalt guter Gebrauchseigenschaften des Ballonbauteiles zum Ziel haben. Es besteht also weiterhin ein Bedarf an neuen Materialien für die Herstellung von Ballonkathetern mit verbesserten Eigenschaften.

Aufgabe der vorliegenden Erfindung ist es, einen oder mehrere Nachteile des Standes der Technik zu vermindern oder zu vermeiden.

Die vorliegende Erfindung löst diese Aufgabe durch Bereitstellung eines Katheters mit einem dilatierbaren Ballon, dadurch gekennzeichnet, dass die primäre Ballonwand aus einem Material gebildet ist, welches ein Polyamid/Polyvinylpyrrolidon (PA/PVP)-Polymergemisch umfasst oder daraus besteht.

Durch die Zugabe von Polyvinylpyrrolidon (PVP), bevorzugt als oligomerer bis makromolekularer Zusatz zu Polyamiden, wird ein Polymergemisch oder -blend erzeugt, welches für die Herstellung der primären Ballonwand des dilatierbaren Ballons eines Katheters wie auch für Bauteile des Schaftes eingesetzt werden kann. Der erhaltene Ballon bzw. die erhaltenen Bauteile zeichnen sich durch eine Steigerung der Verformungsgrenzen aus, sowie durch eine Verzögerung der Kristallisation, eine Zunahme an Elastizität, bzw. einer in der Regel geringen Abnahme des E-Moduls und führt zu einer Erhöhung des Glaspunktes im trockenen Zustand der Probe. Dieses Eigenschaftsspektrum ist technisch von hohem Interesse, weil die Zunahme an Elastizität die Verformungsgrenzen im Blasprozess erweitert und durch Steigerung der Orientierung des Polymeren einen höheren Verstärkungseffekt erreichen lässt. Die Verringerung des E-Modules des Materials kann somit bei geeignetem Design durch einen Gewinn an Orientierung des Polymeren kompensiert werden. Dies führt zu einer deutlichen Verbesserung der Gebrauchseigenschaften des Ballons.

Die verzögerte Kristallisation nach der Extrusion führt zu verbesserten und toleranteren Umformungsgrenzen, dies ist insbesondere für die Ballonverformung und deren Prozessstabilität vorteilhaft. So sind z. B. kleinere Hälse bei der Formung erreichbar, bzw. höher ausgereckte Bereiche des geformten zylindrischen Teiles des Ballons. Es werden Ballone mit flexibleren Konen erhalten, da die Elastizität erhöht ist und die Kristallisationsneigung bei thermischer Behandlung verringert ist. Durch die weicheren Konen des Ballons wird beispielsweise ein verbessertes Rückfaltvermögen nach Inflation des Ballonbauteils ermöglicht. Die Erhöhung der Verformungsgrenzen erzeugt die Möglichkeit für den zylindrischen Bereich des Ballons eine höhere Verstreckung zu erreichen und somit einer Reduzierung des E-Moduls sowie ggf. auch einer gesteigerten Compliance des Ballonbauteils entgegenzuwirken da eine höhere Orientierung des streckgeformten Bauteiles erreicht werden kann.

Die Kristallisation des Blends ist primär zeitlich verzögert, sie kann somit durch geeignete Verfahrenstechnik trotzdem zu einem Maximum gesteigert werden. In diesem Falle wird beobachtet, das z. B. die Polymermatrix PA12 bei Molekulargewichten entsprechend kommerziellen PVP Type K30 ab einer Konzentration von ca. 3% Gewichtszusatz unter Kristallisationsbedingungen die Diffusion des PVP Zusatzes in Randbereiche des Bauteiles erfolgt, wie unter Umständen auch in ausgeschiedene Phasen beobachtet wird. Balloonbauteile welche aus einem Blend von PA12/PVP aufgebaut sind können unter ungünstigen Verfahrensbedingungen nach Kristallisation zur Ausbildung von punktförmig versagenden Bereichen, sogenannten "pin holes" neigen. Die Entwicklung solcher Bauteile sollte somit eine molekulargewichtsabhängige Grenzkonzentration beachten. Im Falle von "Polymerscharnieren", die ein sehr robustes Bruchverhalten des Polymers benötigen, kann eine sehr ausgeprägte Mikrorissbildung aufgrund dieser Phasenseparation des Polymers, welche z. B. für PA12 durch Blendung mit der kommerziellen PVP Type K90 bereits bei Konzentrationen von 3% Gewichtszusatz beobachtet wird, durchaus vorteilhaft sein. Hiermit sind dann z. B. Katheter denkbar, die auf faltenden, bzw. lokal knickenden Elementen beruhen und trotzdem eine sehr geringe Bruchneigung an den Bewegungsstellen höchster Verformung aufweisen.

Die Ballone weisen in trockenem Zustand einen erhöhten Glaspunkt auf und neigen somit während trockener Lagerung und trockener thermischer Behandlung zu sehr geringer Relaxation. In ETO-Sterilisationszyklen wurde trotz des erhöhten Feuchtigkeitsaufnahmevermögens an gefertigten Balloonbauteilen keine auffällig erhöhte Relaxation beobachtet. Nach der Formung und Orientierung des Polymergemisches wird PVP an der Phasengrenze angereichert. Während Polyamide an der Oberfläche meist eine geringe Reaktivität und Benetzbarkeit aufweisen, zeigt die Anwesenheit von PVP an der Oberfläche eine haftvermittelnde, benetzende Wirkung wie auch einen hydrophilierenden Effekt. So wird z. B. nach der Formung von Ballonbauteilen im Wasserbad eine komplette Benetzung der Oberfläche mit Wasser beobachtet sowie eine geringere Schlüpfrigkeit haptisch festgestellt. Da PVP sowohl lipophile, als auch polare Wechselwirkungen erlaubt und naturgemäß ein Vinylpolymer darstellt, lassen sich Ballonbaugruppen aus PA/PVP-Polymergemischen im Gegensatz zu reinen Polyamiden sehr gut durch Acrylate kleben. Diese Verbindungen erweisen sich als beständiger gegen Alterung und Delamination. Die Ballonoberfläche zeigt bereits ohne zusätzliche Beschichtung eine bessere Haftung zu polaren, wie auch unpolaren, physikalisch haftenden Beschichtungen, wie z. B. Wirkstoff-freisetzenden Deckschichtungen. Da die Oberfläche durch wässrige Systeme wesentlich besser benetzt wird, lässt sich ein blasenfreieres Befüllen von Schläuchen oder Kavitäten einfacher bewerkstelligen. Dies ist in der Medizintechnik in vielen Bereichen essentiell - z. B. bei Oxigenatoren - ist aber auch für Katheter mit besonders großen und/oder langen Ballonen durchaus hilfreich und je nach Anwendung von sicherheitsrelevantem Interesse. Dilatationen im Bereich des linken Atriums, angrenzenden Pulmunarvenen, dem linken Ventrikel und im Bereich der Aortenklappe müssen aufgrund der Dimensionen mit relativ großen Balloonbauteilen durchgeführt werden. Diese sollten typischer Weise blasenfrei präpariert werden, da Sie im Falle des Versagens, potentiell Gasblasen freisetzen und in Hirn- und Coronargewebe Mikroembolien verursachen können.

Entsprechend der vorhergehenden Erklärungen der veränderten mechanischen Eigenschaften, wie auch der beobachteten Grenzflächenphänomene sind neben Kathetern mit einem dilatierbaren Ballon auch andere Katheteranwendungen mit vorteilhafter Umsetzung des PA/PVP Blends von der vorliegenden Erfindung umfasst.

Die Erfindung bezieht sich maßgeblich auf einen Katheter mit einem dilatierbaren Ballon. Darunter können Katheter verstanden werden, die dazu geeignet sind, einen Stent zu applizieren und dafür einen dilatierbaren Ballon aufweisen. Darunter werden aber auch Katheter verstanden, deren dilatierbarer Ballon direkt therapeutisch eingesetzt werden kann, beispielsweise im Rahmen einer Ballondilatation zur Aufweitung von Gefäßverengungen. Grundsätzlich kann für den erfindungsgemäßen Katheter jedes bekannte Kathetersystem verwendet werden, bevorzugt jedes Kathetersystem, welches einen dilatierbaren Ballon umfasst.

Insbesondere betrifft die Erfindung einen Katheter mit einem Innenschaft, an dem am distalen Ende ein dilatierbarer Ballon befestigt ist, der in einem nicht-expandierten, deflatierten Zustand an einer Außenoberfläche des Innenschaftes zumindest teilweise anliegt. Katheter der vorgesehenen Art können neben einem Innenschaft und dem dilatierbaren Ballon auch einen Außenschaft aufweisen, der wenigstens bis zu einem proximalen Ende des Ballons reicht und mit diesem fluiddicht verbunden ist. Zwischen Innen- und Außenschaft des Katheters ist üblicherweise eine in Längsrichtung des Katheters von seinem proximalen Ende bis ins Innere des Ballons reichende Fluidleitung vorgesehen, die sich beispielsweise daraus ergibt, dass der Außenschaft einen Innendurchmesser besitzt, der größer ist, als ein Außendurchmesser des Innenschaftes.

Im Inneren des Innenschaftes ist ein vom Innenschaft eingeschlossener, sich in Längsrichtung des Innenschaftes erstreckender Hohlraum als Lumen vorgesehen. Dieses Lumen dient beispielsweise der Aufnahme eines Mandrins oder eines Führungsdrahtes. Katheter und Führungsdraht sind dann beispielsweise so ausgebildet, dass der Führungsdraht an der distalen Spitze des Katheters austreten kann und vom proximalen Ende her zu steuern ist. Der Führungsdraht wird zum Beispiel mit Hilfe von Steuermitteln so ausgelenkt, dass er auch in abzweigende Blutgefässe leicht einzuführen ist. Der Ballonkatheter kann dann entlang des Führungsdrahtes nachgeschoben werden.

Unabhängig von der Art des Katheters, insbesondere hinsichtlich der Gestaltung der Führungsmittel, weisen die erfindungsgemäßen Katheter an ihrem distalen Ende den bereits erwähnten, dilatierbaren Ballon auf. Während des Einführens des Katheters ist der Ballon komprimiert und liegt eng am Innenschaft des Katheters an. Durch Inflatieren des Ballons mit einem Fluid kann dieser expandiert oder dilatiert werden. Dieses Expandieren des Ballons geschieht, sobald der Bereich des Katheters mit dem dilatierbaren Ballon bis zu der bestimmungsgemäßen Position geführt ist. Durch das Expandieren des Ballons kann ein Stent appliziert werden oder es wird eine Oberfläche des Ballons an eine Gefäßwand anlegt. Dies geschieht beispielsweise zu dem Zweck, Gefäßverengungen (Stenosen) mittels des Ballons des Katheters zu weiten.

Der erfindungsgemäße Katheter zeichnet sich dadurch aus, dass die primäre Ballonwand des dilatierbaren Ballons aus einem Material gebildet ist, welches ein PA/PVA-Polymergemisch umfasst oder daraus besteht. Unter der primären Ballonwand wird die Wand verstanden, die das Lumen des Ballons umschließt und somit den Ballon bildet. Die primäre Ballonwand bildet die Basis des Ballons bzw. dessen Wandung auf die ggf. später zusätzliche Materialien und/oder Beschichtungen aufgetragen werden können. Die primäre Ballonwand umfasst ausdrücklich nicht solche zusätzlichen oder nachträglich hinzugefügten Beschichtungen, die auf der Innen- und/oder Außenoberfläche der primären Ballonwand aufgetragen werden können oder aufgetragen sind.

Das PA/PVP-Polymergemisch bezeichnet ein Polymerblend enthaltend oder bestehend aus den beiden Polymertypen Polyamid (PA) und Polyvinylpyrrolidon (PVP). Unter einem Polymergemisch oder Polymerblend wird dabei eine physikalische Mischung der Polymere verstanden und liegt als makroskopisch homogene Mischung der verschiedenen Polymeren vor. Dabei findet zwischen den unterschiedlichen Polymeren keine chemische Reaktion statt. In der Literatur wird diskutiert, dass es zu einer Bildung eines Komplexes zwischen Polyamidbindungen des PA und dem Pyrrolring des PVP kommen kann. Hergestellt werden Polymermischungen oder -Blends durch mechanische Vermischung von geschmolzenen Polymeren, wobei sich ein homogenes Material ergibt. Insbesondere können Polymergemische dadurch erreicht werden, dass das eine Polymer in fester oder flüssiger Form der Schmelze des anderen Polymeren zugesetzt und mitaufgeschmolzen wird. Beim Abkühlen der gemischten Schmelze bleiben die unterschiedlichen Polymerketten gemischt und es wird davon ausgegangen, dass bei genügend intensiver Mischung und auch genügend geringer Dosierung eine physikalische Mischung beider Polymere erreicht wird, die auch dauerhaft erhalten bleibt.

Das PA/PVP-Polymergemisch besteht aus einem Polyamid- und einem Polyvinylpyrrolidonanteil und kann ggf. weitere Bestandteile wie Lösungsmittel und/oder Weichmacher aufweisen.

Eine interessante Variante kann wegen der benetzenden Wirkung auch der Nutzen als verarbeitungshilfsmittel zur Dispergierung von Füll- oder Verstärkungsstoffen sein. So ist zum Beispiel eine Exfoilierung von Schichtsilikaten (Clays) in wässriger Lösung unter Anwesenheit von PVP möglich. Über einen Sprühtrocknungsprozess lässt sich ein rieselfähiges Pulver erzeugen, welches der Compoundierung mittels einer gravimetrisch dosierenden Schüttelrinne zugeführt werden kann. Da die Viskositätszunahme der Clays in wässriger Lösung erheblich ist und zu verfahrenstechnischen Problemen führen kann, ist als Verfahrensvariante die Exfoilierung des Clays in einem wasserhaltigen Gemisch, z. B. einem Alkohol Wasser Gemisch genutzt werden. Somit ist eine strukturelle Verstärkung des Polymers zu einem Schichsilkatverstärken Material (Nanaocomposit) und die vorteilhafte Beeinflussung dessen Kristallinität über überschüssiges PVP welches sich in der Polyamid basierten Polymermatrix zu lösen vermag möglich. Der Gehalt von Schichsilikat sollte hierbei geringer als 7% (Gewicht) und ca. 1-5%(Gewicht) an PVP sein.

Bevorzugt bilden die Gewichtsanteile der Polyamid- und der Polyvinylpyrrolidonanteile zusammen 100 Gew.% des PA/PVP-Polymergemisches.

Der Gewichtsanteil des PVP im PA/PVP-Polymergemisch beträgt immer < 10 Gew.% bezogen auf das Gesamtgewicht des PA/PVP-Polymergemisches. Bevorzugt beträgt der Gewichtsanteil des Polyvinylpyrrolidonanteils im PA/PVP-Polymergemisch 0,01 bis 7 Gew.% bezogen auf das Gesamtgewicht des PA/PVP-Polymergemisches, besonders bevorzugt 0,5 bis 5 Gew.%, ganz besonders bevorzugt 1 bis 3 Gew.%. Im PA/PVP-Polymergemisch wird bevorzugt Polyvinylpyrrolidon verwendet mit einer mittleren Molmasse von oligomeren Verbindungen bis hin zu bis Verbindungen mit einer mittleren Molmasse von 2.500.000 g/mol. Der Polyvinylpyrrolidonanteil des PA/PVP-Polymergemisches kann ein Polyvinylpyrrolidon mit einem K-Wert von 20 bis 100 enthalten oder daraus bestehen, bevorzugt PVP mit einem K-Wert (K-Wert nach Fikentscher) von 30 bis 90, besonders bevorzugt PVP mit einem K-Wert von 30, 60 oder 90. Es kann sich als vorteilhaft erweisen, das Molekulargewicht von PVP durch eine geeignete Verfahrenstechnik gezielt zu verringern, wobei bevorzugt hochmolekulare Bestandteile abgebaut werden sollen. Diese Reduktion des Molekulargewichtes ist zum Beispiel durch die Einwirkung von intensiver mechanischer Scherung (Ultraturrax - Dissolver) wie auch durch intensiven Ultraschalleinfluss auf gelöstes PVP realisierbar.

Der Gewichtsanteil des Polyamid im PA/PVP-Polymergemisch beträgt immer > 10 Gew.% bezogen auf das Gesamtgewicht des PA/PVP-Polymergemisches. Der Polyamidanteil des PA/PVP-Polymergemisches kann lediglich ein bestimmtes Polyamid beinhalten oder eine Mischung verschiedener Polyamide. Bei dem Polyamid kann es sich um ein Homopolymer handeln oder um ein Copolymer. Bevorzugt enthält oder besteht der Polyamidanteil des PA/PVP-Polymergemisches ein Polyamid, welches ausgewählt ist aus PA5, PA6, PA7, PA8, PA9, PA10, PA11, PA12, PA13, PA14 und/oder PA15 oder einem Copolymer enthaltend mindestens ein Monomer der vorgenannten Art, bevorzugt aus PA6, PA7, PA8, PA9, PA10, PA11 und/oder PA12 oder einem Copolymer enthaltend mindestens ein Monomer der vorgenannten Art, besonders bevorzugt ist das Polymer ein PA12.

Alternativ können zum Beispiel anstatt von Polyamiden Polyurethane oder Peptide in den erfindungsgemäßen Polymergemischen zum Einsatz gebracht werden.

Alternativ zu PVP können in den erfindungsgemäßen Polymergemischen beispielsweise PVP-Copolymere eingesetzt werden (z. B. die Produktfamilie unter den Handelsnahmen Luvitec).

Aus der Literatur kann entnommen werden, dass die Wasserstoffbrückenbindung der Amidbindung durch PVP gemindert wird, welche vermutlich auch mit einer Komplexbildung zu dem PVP verbunden ist. Somit sind sowohl aliphatische, wie auch aromatische Polyamidtypen in ihrem Eigenschaftsspektrum veränderbar.

Das PA/PVP-Polymergemisch des erfindungsgemäßen Katheters kann nach bekannten Verfahren hergestellt und/oder kompoundiert werden. Bevorzugt wird das PA/PVP-Polymergemisch durch Kompoundierung hergestellt, da hier sowohl eine schonende, wie auch angepasste Scherung zur Durchmischung realisierbar ist. Eine gewichtskontrollierte Dosierung der Einzelkomponenten ist bei diesem Verfahren Stand der Technik. Vorzugsweise wird das Polymergemisch in einem Doppelschneckencompounder mit gravimetrischer Dosierung für Granulat und das PVP in pulverförmiger Konsistenz hergestellt. Dabei wird bevorzugt das getrocknete, feindisperse PVP z. B. über eine Schüttelrinne einer PA-Schmelze zugeführt. Die Verwendung einer Schutzgasatmosphäre wie auch einer Vakuumentgasung der plastifizierten und gemischten Schmelze vor der Strangbildung ist vorteilhaft.

Im erfindungsgemäßen Katheter können neben dem Ballon noch weitere Bestandteile des Katheters aus einem Material gefertigt sein, welches ein PA/PVP-Polymergemisch umfasst oder daraus besteht.

Der erfindungsgemäße Katheter kann mindestens auf Teilen der Außenoberfläche des dilatierbaren Ballons eine Wirkstoff-freisetzende Beschichtung und/oder Kavitätenfüllung aufweisen. Die Beschichtung kann auch die gesamte Außenoberfläche des Ballons bedecken. Eine Beschichtung im Sinne der Erfindung ist eine zumindest abschnittsweise Auftragung der Komponenten der Beschichtung auf die Außenoberfläche des dilatierbaren Ballons des Katheters. Dabei wird die Oberfläche des Ballons als Außenoberfläche bezeichnet, die während des klinischen Einsatzes für gewöhnlich mit der Gefäßwand kontaktierbar ist oder in Kontakt gebracht wird. Vorzugsweise wird die gesamte Außenoberfläche des Ballons von der Beschichtung bedeckt. Eine Schichtdicke liegt vorzugsweise im Bereich von 1 nm bis 100 µm, besonders bevorzugt im Bereich von 300 nm bis 50 µm. Die Beschichtung kann direkt auf die Ballonoberfläche aufgetragen werden. Die Verarbeitung kann nach Standardverfahren für die Beschichtung erfolgen. Es können einschichtige, aber auch mehrschichtige Systeme (zum Beispiel so genannte base coat -, drug coat - oder drug comprising top coat - Schichten) erstellt werden. Die Beschichtung kann unmittelbar auf dem Ballonkörper aufgebracht sein oder es können weitere Schichten dazwischen vorgesehen sein. Alternativ oder zusätzlich kann der Katheter eine Kavitätenfüllung aufweisen. Die Kavität befindet sich in der Regel an der Außenoberfläche des dilatierbaren Ballons. Verfahren zur Beschichtung von Kathetern und zur Anbringung von Kavitätenfüllungen auf Kathetern sind dem Fachmann bekannt.

Die vorliegende Erfindung bezieht sich auch auf ein Verfahren zur Herstellung des Ballons eines erfindungsgemäßen Katheters, wobei der charakteristische Verfahrensschritt in der Wahl der Temperatur, ggf. in Kombination mit der Wahl des Drucks, liegt bei der die Ballonformung erfolgt. Die anderen Verfahrensschritte sind im Wesentlichen identisch mit den Schritten aus bekannten Verfahren zur Herstellung von Ballonen für Katheter aus Polyamiden, insbesondere aus PA12 oder Pebax und werden hier nicht detailliert wiedergegeben.

Im erfindungsgemäßen Verfahren erfolgt die Ballonformung aus einem PA/PVP-Polymergemisch bei einer Temperatur von ≥ 50°C, bevorzugt bei einer Temperatur von ≥ 80°C. Typischerweise erfolgt die Ballonformung in einem Wasserbad oder über einen Streck-Blas-Prozess.

Die Erfindung bezieht sich auch auf die Verwendung eines Materials, welches das oben genannte PA/PVP-Polymergemisch umfasst oder daraus besteht, zur Herstellung eines dilatierbaren Ballons eines Katheters.

Im Folgenden wird die Erfindung anhand von Ausführungsbeispielen näher verdeutlicht.

### Ausführungsbeispiel:

Das PA/PVP-Polymergemisch wird mit einem Doppelschneckencompounder unter gravimetrischer Dosierung hergestellt. Das Polyamid wurde routinemäßig in einem Umlufttrockner vorkonditioniert. Das PVP wurde zuvor in einem Vakuumtrockenschrank über Nacht getrocknet bei 120°C und einem Druck <50mbar getrocknet.

In einem ersten Versuch wurde ein Doppelschnechencompounder der Firma Coperion mit einem Schneckendurchmesser von 20mm, L/D > 40, gravimetrisch kontrollierter Granulatförderung und einer gravimetrischen Vibrationsrinne für die Zudosierung des pulverförmigen PVP in die PA-Schmelze eingesetzt.

Es wurden zwei verschiedene PVP Typen, K30 und K90 von Bayer, und zwei gravimetrische Dosierungsstufen von 3 Gew.% und 6 Gew.% PVP, jeweils bezogen auf das Gesamtgewicht des PA/PVP-Polymergemisches, geblendet. Die Prozesstemperatur wurde auf ein Temperaturprofil eingestellt welches bei maximal 220°C endet.

Die kompoundierte Schmelze wurde durch ein Wasserbad abgeschreckt und der Strangranulierung zugeführt. Dieses Granulat wird einer intensiveren Trocknung zugeführt als dies für reine Polyamide desselben Typs üblich ist, da das Wasseraufnahmevermögen deutlich erhöht ist. Nach Trocknung erfolgt die Schlauchextrusion welche sich kaum von den typischen Extrusionsbedingungen von Polyamiden unterscheidet. Erfahrungsgemäß sollten allerdings Temperaturen > 220°C vermieden werden.
Sämtliche Versuchsmaterialien zeigten nach erfolgter Extrusion zum Schlauch die Fähigkeit mit ggf. leicht geänderten Prozesstemperaturen zu Ballonen geformt zu werden.

Die mechanischen Eigenschaften der kompoundierten Schläuche führten für die beiden PVP-Typen jeweils zu ähnlichen Eigenschaften in maximaler Bruchdehnung und Zugwert. Der optische Eindruck gestreckter Proben ist jedoch je nach PVP-Typ deutlich unterschiedlich. Die K90 Type zeigte unter den gewählten Verfahrensbedingungen durch die weißlichen Vlieszonen die Ausbildung von Mikrorissstrukturen (crazes) an. Die Schläuche der K30 Type blieben dagegen vollständig transparent. Im Falle dieser Versuchsbedingungen mit der K 30 Type wurden Molekulargewicht wie auch Konzentration geeignet gewählt und somit eine vollständige physikalische Lösung des PVP's in der Polyamidmatrix erreicht.

In der Ballonformung, welche im Wasserbad durchgeführt wurde, mussten die Konditioniertemperaturen verglichen zu unmodifiziertem Polyamid deutlich erhöht werden. Typischer Weise werden Polyamide bei der angewandten Prozesstechnik bei ca. 80°C und Drücken um 40 bar geblasen. Die Ballone aus den PA/PVP-Polymergemischen werden bei einer Temperatur von ca. 95°C bei einem Druck von ca. 40 bar geblasen.

Die geformten PA/PVP-Ballone entwickeln sich "explosionsartig", während sich die reinen Polyamid-Ballone des Standes der Technik weniger abrupt formen. Die frisch entformten Ballone aus dem Wasserbad weisen ein leicht "schlüpfrigeres" Verhalten verglichen zu reinen Polyamid-Ballonen auf.

Die Schläuche aus dem PA/PVP-Polymergemisch lassen sich zu höheren radialen Reckraten ausformen als reine Polyamidballone. Es wurden Ballone mit sehr flexiblen und dünnen Konen erhalten. Die PA/PVP-Ballone zeigten häufig "pin-holes". Dies weist darauf hin, dass die Kompoundierung bei aggressiveren Bedingungen erfolgen kann als bisher realisiert und ggf. auch die Konzentration an PVP noch verringert werden kann. Die "pin-hole"-Häufigkeit wird durch einen zusätzlichen Temperprozess verstärkt. Die Häufung der "pin-holes" und das schlüpfrige Verhalten der PA/PVP-Ballone nach Entformung aus dem Wasserbadprozess lassen darauf schließen, dass das PA/PVP-Polymergemisch bei hohen Temperaturen weiterhin zur Kristallisation und Umstrukturierung der Polymermatrix neigt und PVP aus kristallisierenden Bereichen des Polymers verdrängt wird. Es ist davon auszugehen, dass während des Kristallisationsprozesses von PA12 PVP-Anteile aus der amorphen Matrix ausgesondert, bzw. aus amorphen Bereichen der Matrix verdrängt werden. Diese Beobachtung bestätigt indirekt die Effektivität dieses Additives den Polyamidschlauch nach Extrusion bevorzugt amorph zu halten um erweiterte Verformungsgrenzen des Polymers für den Formungsprozess zu erreichen, wie auch den gewünschten Effekt durch Kristallisation des orientierten Polymers unter erhöhten Temperaturen ein gefestigtes und dimensionsstabileres Bauteil zu ermöglichen.

Es wurden aus Blends von Nylon 12 (Grilamid L25) und 6 Gew.% PVP (K30 Bayer) Ballone mit dem Formdurchmesser von 7.0 mm erhalten. Die PA/PVP-Ballone weisen doppelte Wandstärken um 50µm und eine Durchmessersteigerung bei 6 bis 12 bar von 7,21 mm zu 7.56 mm auf. Es wurden Berstdrücke von ca. 13-14 bar erreicht. Dies deutet auf eine Druckfestigkeit zwischen PA12 und Pebax 7033 hin, wobei interessanter Weise die Compliance deutlich unter der von vergleichbar hergestellten Pebax Ballonen verbleibt.

## Patentansprüche

1. Katheter mit einem dilatierbaren Ballon, **dadurch gekennzeichnet, dass** die primäre Ballonwand aus einem Material gebildet ist, welches ein Polyamid/Polyvinylpyrrolidon (PA/PVP)-Polymergemisch umfasst oder daraus besteht.

2. Katheter nach Anspruch 1, wobei das PA/PVP-Polymergemisch aus einem Polyamid- und einem Polyvinylpyrrolidonanteil besteht, wobei die Gewichtsanteile des Polyamid- und des Polyvinylpyrrolidonanteils zusammen im Wesentlichen 100% ergeben.

3. Katheter nach Anspruch 1 oder 2, wobei der Gewichtsanteil des Polyvinylpyrrolidonanteils im PA/PVP-Polymergemisch 0,01 bis 7 Gew.% beträgt, bevorzugt 0,5 bis 5 Gew.%, besonders bevorzugt 1 bis 3 Gew.%.

4. Katheter nach einem der vorhergehenden Ansprüche, wobei der Polyamidanteil des PA/PVP-Polymergemisches ein Polyamid enthält oder daraus besteht, welches ausgewählt ist aus PA5 bis PA15, bevorzugt aus PA6 bis PA12, besonders bevorzugt ist das Polymer ein PA12.

5. Katheter nach einem der vorhergehenden Ansprüche, wobei der Polyvinylpyrrolidonanteil des PA/PVP-Polymergemisches ein Polyvinylpyrrolidon mit einer mittleren Molmasse von 2.500 bis 2.500.000 g/mol enthält oder daraus besteht.

6. Katheter nach einem der vorhergehenden Ansprüche, wobei der Polyvinylpyrrolidonanteil des PA/PVP-Polymergemisches ein Polyvinylpyrrolidon mit einem K-Wert von 20 bis 100 enthält oder daraus besteht, bevorzugt mit einem K-Wert von 30 bis 90, besonders bevorzugt mit einem K-Wert von 30, 60 oder 90.

7. Katheter nach einem der vorhergehenden Ansprüche, wobei neben dem Ballon noch mindestens ein weiterer Bestandteil des Ballonkatheters aus einem Material gebildet ist, welches ein PA/PVP-Polymergemisch umfasst oder daraus besteht.

8. Katheter nach einem der vorhergehenden Ansprüche, wobei der dilatierbare Ballon auf der Außenoberfläche der primären Ballonwand eine Beschichtung oder Kavitätenfüllung aufweist.

9. Katheter nach einem der Ansprüche 1 bis 8, wobei der Katheter einen Stent trägt.

10. Verfahren zur Herstellung des dilatierbaren Ballon eines Katheters nach einem der Ansprüche 1 bis 9, **dadurch gekennzeichnet, dass** die Ballonformung bei einer Temperatur von ≥ 50°C erfolgt, bevorzugt bei einer Temperatur von ≥ 90°C.

11. Verfahren nach Anspruch 10, wobei die Ballonformung im Wasserbad erfolgt.

12. Verwendung eines Materials, welches ein PA/PVP-Polymergemisch umfasst oder daraus besteht, zur Herstellung eines dilatierbaren Ballons eines Katheters.

## Claims

1. A catheter comprising a dilatable balloon, **characterized in that** the primary balloon wall is produced from a material that comprises or consists of a polyamide/polyvinylpyrrolidone (PA/PVP) polymer mixture.

2. The catheter according to claim 1, wherein the PA/PVP polymer mixture comprises a polyamide content and a polyvinylpyrrolidone content, with the weight percentages of the polyamide and polyvinylpyrrolidone contents together totaling substantially 100%.

3. The catheter according to claim 1 or 2, wherein the weight percent of the polyvinylpyrrolidone content in the PA/PVP polymer mixture is 0.01 to 7% by weight, preferably 0.5 to 5% by weight, and particularly preferred 1 to 3% by weight.

4. The catheter according to any one of the preceding claims, wherein the polyamide content of the PA/PVP polymer mixture comprises or consists of a polyamide that is selected from PA5 to PA15, preferably PA6 to PA12, and the polymer is most preferably a PA12.

5. The catheter according to any one of the preceding claims, wherein the polyvinylpyrrolidone content of the PA/PVP polymer mixture comprises or consists of a polyvinylpyrrolidone having an average molecular weight of 2,500 to 2,500,000 g/mol.

6. The catheter according to any one of the preceding claims, wherein the polyvinylpyrrolidone content of the PA/PVP polymer mixture comprises or consists of a polyvinylpyrrolidone having a K-value of 20 to 100, preferably a K-value of 30 to 90, and most preferably a K-value of 30, 60, or 90.

7. The catheter according to claim 1, wherein in addition to the balloon, at least one further component of the balloon catheter is produced from a material comprising or consisting of a PA/PVP polymer mixture.

8. The catheter according to any one of the preceding claims, wherein the dilatable balloon comprises a coating or cavity filling on the outer surface of the primary balloon wall.

9. The catheter according to one of the claims 1 to 8, wherein the catheter has a stent.

10. A method for producing the dilatable balloon of a catheter according to one of the claims 1 to 9, **characterized in that** the balloon shaping is carried out at a temperature of ≥ 50°C, and preferably at a temperature of ≥ 90°C.

11. The method according to claim 10, wherein the balloon shaping is carried out in a water bath.

12. Use of a material comprising or consisting of a PA/PVP polymer mixture for the production of a dilatable balloon of a catheter.

## Revendications

1. Cathéter doté d'un ballon dilatable, **caractérisé en ce que** la paroi de ballon primaire est constituée d'un matériau, lequel comprend un mélange de polymères à base de polyamide/polyvinyl-pyrrolidone (PA/PVP) ou en est constitué.

2. Cathéter selon la revendication 1, dans lequel le mélange de polymères PA/PVP est constitué d'une partie polyamide et d'une partie polyvinylpyrrolidone, où les proportions en poids de la partie polyamide et de la partie polyvinylpyrrolidone sont dans l'ensemble sensiblement égales à 100 %.

3. Cathéter selon la revendication 1 ou 2, dans lequel la proportion en poids de la partie polyvinylpyrrolidone dans le mélange de polymères PA/PVP est de 0,01 à 7 % en poids, de préférence de 0,5 à 5 % en poids, de manière particulièrement préférée de 1 à 3 % en poids.

4. Cathéter selon l'une des revendications précédentes, dans lequel la partie polyamide du mélange de polymères PA/PVP contient un polyamide ou en est constituée, lequel est choisi parmi les PA5 à PA15, de préférence parmi les PA6 à PA12, idéalement le polymère est un PA12.

5. Cathéter selon l'une des revendications précédentes, dans lequel la partie de polyvinylpyrrolidone du mélange de polymères ¨PA/PVP contient une polyvinylpyrrolidone avec une masse moléculaire moyenne de 2 500 à 2 500 000 g/mol, ou en est constituée.

6. Cathéter selon l'une des revendications précédentes, dans lequel la partie de polyvinylpyrrolidone du mélange de polymères ¨PA/PVP contient une polyvinylpyrrolidone avec une valeur K de 20 à 160, ou en est constituée, de préférence avec une valeur K de 30 à 90, de manière particulièrement préférée avec une valeur K de 30, 60 ou 90.

7. Cathéter selon l'une des revendications précédentes, dans lequel, mis à part le ballon, il y a encor au moins une autre composante du cathéter à ballon constituée d'un matériau, lequel comprend un mélange de polymères PA/PVP ou en est constituée.

8. Cathéter selon l'une des revendications précédentes, dans lequel le ballon dilatable présente un revêtement ou un remplissage de cavités sur la surface extérieure de la paroi de ballon primaire.

9. Cathéter selon l'une des revendications 1 à 8, où le cathéter porte un stent.

10. Procédé de fabrication d'un ballon dilatable d'un cathéter selon l'une des revendications 1 à 9, **caractérisé en ce que** la formation du ballon a lieu à une température ≥ 50 °C, de préférence à une température ≥ 90 °C.

11. Procédé selon la revendication 10, dans lequel la formation du ballon a lieu dans un bain d'eau.

12. Utilisation d'un matériau, lequel comprend un mélange de polymères PA/PVP, ou en est constitué, pour la fabrication d'un ballon dilatable d'un cathéter.
